# EUROPEAN PATENT APPLICATION

(11) **EP 4 108 292 A1**
(43) Date of publication of application: **28.12.2022**
(21) Application number: 22177199.1
(22) Date of filing: 08.05.2013
(51) Int. Cl.: A61N 1/36

(54) **METHOD AND NEUROPROSTHETIC DEVICE FOR MONITORING AND SUPPRESSION OF PATHOLOGICAL TREMORS THROUGH NEUROSTIMULATION OF THE AFFERENT PATHWAYS**

(62) Divisional of application: 13382169.4
(71) Applicant: Consejo Superior De Investigaciones Científicas (CSIC), 28006 Madrid (ES); Georg-August-Universität Göttingen Stiftung Öffentlichen Rechts Universitätsmedizin, 37075 Göttingen (DE)
(72) Inventor: ROCON DE LIMA, Eduardo, 28500 Arganda del Rey (Madrid) (ES); PONS ROVIRA, Jose Luis, 28500 Arganda del Rey (Madrid) (ES); FARINA, Dario, 37075 Göttingen (DE); DOSEN, Strahinja, 37075 Göttingen (DE)
(74) Representative: Ungria López, Javier

(57) **Abstract**

Method and neuroprosthetic device for monitoring and suppression of pathological tremors in a user through the neurostimulation of the afferent pathways to the brain, which comprises wearable elements placed over the parts of the body affected by tremor, wherein said wearable elements (1) have bioelectric sensors (3) generating a first signal characterization of tremor, biomechanical sensors (4) generating a second signal characterization of tremor, a programmable electronic device (9) comprised of a control, acquisition and processing module of the first and second characterization signals, and a signal generator for the afferent stimulation based on the first and second signal characterization and stimulation electrodes (8) which transmit the neuromodulation signals to the afferent pathways projecting into the central nervous system to modulate the activity of the neural structures responsible for tremor generation.

## Description

### OBJECT OF THE INVENTION

The present invention relates to a method and a neuroprosthetic device for monitoring and suppression of pathological tremor in a user via the stimulation of peripheral afferent pathways. The resulting afferent inflow projects into the central nervous system (spinal cord and brain) where it modulates the activity of the neural structures responsible for generating/maintaining pathological tremor. Therefore, with this method and device, functional compensation (i.e., attenuation) of the pathological tremor by the stimulation of the afferent pathways is achieved, in particular in the upper limbs.

The main fields of application of the present invention are the material and electronic equipment within the orthoprosthetic and rehabilitation industries.

### BACKGROUND OF THE INVENTION

Pathological tremor can be defined as a strong involuntary oscillatory movement of a body part. Tremor is the most common movement disorder and its incidence and prevalence are increasing with the aging of the world population, affecting 15% of people aged between 50 and 89 years. The most common are tremors caused by the two neurodegenerative diseases: Parkinson's disease and essential tremor. Although the disorder is not life-threatening, more than 65% of population suffering from upper limb tremor reports serious difficulties in performing their activities of daily living (ADL), greatly decreasing their independence and quality of life. The most common treatments are drug therapy or surgery. Patents and references described in this document are the current techniques for the treatment of tremor. However, 25% of patients do not benefit from any of these treatments, and this calls for the creation of alternative treatments to tremor.

The pathological tremors - simply referred to as tremors in the remainder of the document - arise due to various conditions. As a result, it is very difficult to differentiate tremors according to their etiology. Because their exact underlying mechanisms have not yet been clarified, none of them is fully understood. In the state-of-the-art, the tremors are treated with drug therapy or surgery, which may include thalamotomy or pallidotomy, or more commonly nowadays, thalamic stimulation (DBS). Unfortunately, both alternatives have significant drawbacks: drugs often have side effects, and show a decrease in effectiveness over the years of use (Olanow, CW et al. (2000) Preventing levodopa-induced dyskinesias, Ann Neurol 47 , S167-S176; discussion S176-168), while the DBS is associated with an increased risk of intracranial hemorrhage (~ 4% of patients) (Kleiner-Fisman, G, et al. (2006). Subthalamic nucleus deep brain stimulation: Summary and meta-analysis of outcomes, Mov Disord 21, S290-S304) and psychiatric manifestations (Piasecki, SD et al. (2004) Psychiatric Complications of deep brain stimulation for Parkinson's disease, J Clin Psychiatry 65, 845-849), moreover, the percentage of eligible patients is very low (Perlmutter, JS et al. (2006) Deep brain stimulation, Annu Rev Neurosci 29, 229-257), for example, only 1.6 to 4.5% of the Parkinson's disease patients (Morgante, L, et al. (2007) How many parkinsonian patients are suitable candidates for deep brain stimulation of subthalamic nucleus? Results of a questionnaire, Parkinsonism Relat Disord 13, 528-531). Furthermore, the mechanisms accounting for the alleviation of tremor symptoms by drugs, thalamotomy or DBS are unknown, hampering the refinement of the existing treatment forms, and the development of novel ones.

Recently, the state of the art has demonstrated the feasibility of managing upper limb tremors with biomechanical loading, applied either through robotic exoskeletons, or transcutaneous neurostimulation. This approach, on the contrary to pharmacotherapy or surgery, suppresses tremors by the modification of limb biomechanics, and not by targeting their site of origin. In spite of attaining a systematic attenuation of moderate and severe tremors, there are a number of limitations in wearability, comfort, and selectivity (Rocon, E, et al. (2007) Design and validation of a rehabilitation robotic exoskeleton for tremor assessment and suppression, IEEE Trans Neural Syst Rehabil Eng 15, 367-378). These techniques are protected by various patents: ES200301767, ES200402966, US5562707, and US6959215 US7643882B2.

Recent experiments with subthreshold (afferent) transcutaneous stimulation carried out in the framework of TREMOR project (ICT-2007-224051) showed significant tremor attenuation in a number of patients (unpublished results). This finding is in agreement with a recent work on restoration of locomotion in rodent models of Parkinson Disease through spinal cord stimulation (Fuentes, R, Peterson, et al. (2009) Spinal cord stimulation restores locomotion in animal models of Parkinson's disease, Science 323, 1578-1582). Therefore, current evidences support the idea that stimulation of the afferent pathways may alleviate the symptoms of tremors, probably by a disruption of low-frequency synchronization in the corticobasal ganglia circuits (Fuentes, R, et al. ( 2010) Restoration of locomotive function in Parkinson's disease by spinal cord stimulation: mechanistic approach, Eur J Neurosci 32, 1100-1108).

Within the state of the art, there are several patents that describe different techniques of stimulation but none protects the concept of this invention. The patent US20060217781A1 (Systems and methods for treating disorders of the central nervous system by the modulation of brain networks) describes a series of methods and systems for the treatment of brain disorders through the neuromodulation of brain structures. The patent describes a number of methods for implementing neuromodulation (electrical, optical, magnetic or chemical stimulation) of the structures of the brain associated with the disease. Patent US20060212089 (Device for the desynchronization of neuronal brain activity) focuses on the description of a device for the desynchronization of brain areas. Importantly, all of the proposed techniques are focused on the direct stimulation of the brain and not on the stimulation of peripheral afferent pathways (sensory nerves), as described in this patent. The patent application US2011184489 entitled "Method of Treating Parkinson's Disease and Other Movement Disorders" describes a method for the treatment of disorders associated with Parkinson's disease based on electrical stimulation of the spinal cord. Patent WO2010141155A1 entitled "Selective neuromodulation using energy-efficient waveforms" describes methods for selectively neuromodulation in mammals.

The patent application WO2005122894 "Method and electronic computing device for suppressing and evaluating tremors and spastic movements in relation to input and control peripherals" describes a method and device for discerning the tremor movement generated by neurological disorders.

### Disclosure of the invention

The present invention discloses a method and a neuroprosthetic device for monitoring and suppression of pathological tremor in a user via neurostimulation of the peripheral afferent pathways (sensory nerves). This invention is employed as an alternative treatment to the reduction of tremor in patients suffering from the pathologies that generate tremor, in particular Essential tremor and Parkinson Disease. It industrial application takes the form of a neuroprosthesis (implanted or transcutaneous) to reduce the tremor through the neuromodulation of the afferent pathways. The final product therefore is a neuroprosthesis, integrating all the electronics for the control, acquisition and processing of tremor variables acquired by bioelectrical sensors (e.g., EMG) or biomechanical movement sensors (e.g., inertial measurement units). This electronics is preferably integrated into a textile substrate to be worn on the human upper limb, with a set of electrodes (implantable or transcutaneous) for neurostimulation. The neuroprosthetic device, in addition to suppressing tremor, may be a tremor-monitoring tool during the patient's daily life. Furthermore, the proposed method allows adapting automatically the neuromodulation strategy to the different conditions of tremor movement in each patient.

A first object of the present invention is a neuroprosthetic device for monitoring and suppression of pathological tremor in a user via stimulation of the afferent pathways. This device is preferably portable. This concept could either replace or complement the traditional pharmacotherapeutic treatment of tremor. The stimulation is closed loop controlled by a series of sensors that quantify the tremor of the person. The sensor data are then processed and used to define adequate levels and timing of the stimulation.

As a result, the neuroprosthetic device comprises at least one part worn by the user and located on the part of the body affected by tremor. The part worn by the user may be preferably a textile garment and placed on the user's arm or forearm, but it could also be placed on the other areas of the body. Each of the wearable elements comprises:
- At least one bioelectrical sensor that generates a bioelectrical characterization of tremor;
- At least one biomechanical sensor that generates a biomechanical characterization of tremor;
- A programmable electronic device comprising at least one module for the control, acquisition and processing of the electrical and biomechanical signal characterizations, and an electric signal generator for generating a signal of the afferent stimulation from the electrical and/or biomechanical characterization signals and,
- At least one stimulation electrode integrated into the wearable textile that transmits the signal for the stimulation to the afferent pathways.

In a particular implementation of the invention the neuroprosthetic device comprises at least one electroencephalography sensor that measures brain activity and generates a signal that characterizes the intention of the user to move the limb affected by tremor.

In another particular implementation of the invention, the device comprises an external device connectable to a programmable electronic device with an interface for accessing the functionalities of at least one wearable element. In a more particular implementation of the invention, the target programmable electronic device can be a computer, a tablet, a smartphone or a similar device. It is also envisaged that in another particular implementation of the invention, the external device comprises means for displaying measurements taken by the neuroprosthetic device based on the acquired signals, a processor for analysis and generation of diagnostics and reports of tremor and a memory to store the measurements performed by the neuroprosthetic device.

In another particular implementation of the invention, the at least one stimulation electrode is an electrode selected between an implanted electrode (e.g., intramuscular wire electrode) and a transcutaneous electrode.

In another particular implementation of the invention, at least one bioelectric sensor is selected among a classic multichannel bipolar EMG, high-density electromyography sensors (hdsEMG), electroencephalography sensors (EEG), or a combination of those, measuring electrical activities of the muscles and brain and characterizing the pathological tremor and/or voluntary movement intentions of the user.

In another particular implementation of the invention, at least one biomechanical sensor is an inertial sensor selected from gyroscopes, accelerometers, magnetometers or a combination of those.

In another particular implementation of the invention, at least one electromyography sensor is a multichannel thin film electrode.

In another particular implementation of the invention, at least one stimulation electrode is a thin film multi-channel electrode.

In another particular implementation of the invention, the connection between the programmable electronic device and the external device is selected from a wireless connection or a wired connection.

Therefore, the present invention discloses an ambulatory neuroprosthesis, preferably wearable, to remotely monitor and treat tremor during the user's daily life, where the neuroprosthesis integrates a neural stimulation and a high resolution measurement systems; these two components are integrated into the neuroprosthesis object of this invention. As a result, with the measurement and stimulation systems integrated into the neuroprosthetic device, the system will merge neural and biomechanical information to achieve a correct parameterization of tremor in everyday life, and use this information to control the closed-loop stimulation for tremor suppression. Additionally, this data will be interpreted in order to assess the tremor, and provide both to the neurologist and the individual patient the metrics reflecting the current status and evolution of the tremor. This will facilitate remote monitoring of treatment. In addition, the patient will get more involved in the therapy. It will also allow an objective comparison regarding a long-term drug therapy, which is the current gold standard for the tremor treatment.

The neuroprosthesis concept provides a novel way to manage the involuntary movements in the part of the body affected by tremor. The system will be easy to use and self-contained; this means that the acquisition, control electronics and power supply will be incorporated into the neuroprosthetic device, providing functional treatment of the most common forms of tremor in upper limb, for instance, those caused by Parkinson and essential tremor. It will facilitate the independence of the patients, and maximize their time out of the care centers, saving costs associated with medical care.

A second object of the present invention is a method of monitoring and suppression of pathological tremor in a user via the stimulation of peripheral afferent pathways, utilizing the neuroprosthetic device described above. This method comprises the following steps:
i) Tremor characterization by a plurality of bioelectrical and biomechanical sensors generating a bioelectrical characterization signal and a biomechanical characterization signal wherein these tremor characterization signals comprise at least frequency, phase or amplitude of tremor;
ii) To provide the programmable electronic device with these tremor characterization signals;
iii) To distinguish the tremor from the voluntary movement of the user through a conventional analysis of the bioelectrical characterization signal and the biomechanical characterization signal;
iv) To calculate the neurostimulation control signal from the bioelectrical and biomechanical characterization signals, which is implemented in the module for control, acquisition and processing of the programmable electronic device;
v) To generate the neurostimulation signal through the electronic signal generator (i.e., electrical stimulator) of the programmable electronic device and,
vi) To send the stimulation signals to the plurality of neurostimulation electrodes that will stimulate the afferents pathways.

In a particular implementation of the invention, when the device has at least one electroencephalography EEG sensor which measures the brain activity of the user, the method further comprises the following steps:
- To add to the signals measured in step i) the user's brain activity as captured by at least one EEG sensor and generate a electroencephalography signal that characterizes the intended user movement of the body part affected by the tremor;
- To provide electroencephalography signals, in phase II) to the control, acquisition and processing module of the programmable electronic device;
- Additionally, to use electroencephalography signal in step iv) to calculate the neurostimulation signal to be sent to the neurostimulation electrodes for the activation of the afferents pathways.

In another particular implementation of the invention, stimulation of the afferent pathways disrupts the low-frequency synchronization in the corticobasal ganglia circuits of the user.

In another particular implementation, the afferent inflow generated by the stimulation of the peripheral afferent pathways projects into the central nervous system (spinal cord and brain) where it modulates the activity of the neural structures responsible for generating/maintaining pathological tremor. The latter will result with tremor attenuation.

In another particular implementation of the invention, the analysis employed by the programmable electronic device for discriminating the tremor from the voluntary movement is based on algorithms in the time and frequency domain. More preferably, the algorithm used is the iterative Hilbert transform. An implementation of this type of signal analysis to differentiate trembling from the voluntary movement of the patient is described in the patent application WO2005122894 (Method and electronic computing device for suppressing and evaluating tremors and spastic movements in relation to input and control peripherals). However, one could employ any technique, which allows clear differentiation of the signal generated by a tremor from the signal generated by a voluntary movement of a user that suffers from a neurological pathological condition.

In another particular implementation of the invention, the programmable electronic device sets:
- The parameters of neuromodulation strategies to be executed, and
- The operating mode of the neuroprosthetic device.

In another particular implementation of the invention the calculation of neurostimulation signal in the programmable electronic device is based on an algorithm in the frequency or time domain. More preferably, the algorithm in the frequency or time domain is selected from an IEEE-1057 standard algorithm, a Kalman Filter and Benedict-Bordner filter.

Therefore, in summary, the proposed pathological tremor monitoring and suppression methods consist of two main stages:
The first stage is a strategy for identifying the characteristics of the tremor from information gathered from a variety of biomechanical (inertial sensors) or physiological (EMG or EEG signal) sensors. The features obtained by this strategy are at least the frequency, phase or amplitude of the tremor signal, differentiating the pathological tremor signal from the voluntary signal by means of any digital algorithm or any analog or digital electronic circuit designed for this purpose. Furthermore, information is obtained on the cerebral activity, for example during preparation and initiation of a voluntary movement of the patient through the EEG signal. Additionally, the characteristics obtained from tremor may be used to monitor, track and log tremor during the patient's daily life.

The second stage consists of a control loop for the neuromodulation of afferent pathways, which uses the tremor information obtained in the previous phase, to define the intensity and timing of electrical stimulation of the afferents pathways in order to relieve the symptoms of tremor, preferably, by interrupting the synchronization of tremor-related oscillations at spinal level or brain, thereby preventing the development of tremor. In this context, two possible control strategies are provided that can be implemented by the method and device object of this patent, both based on the neurophysiological tremor origin:
- A first strategy is a desynchronization of the tremorogenic central network by means of afferent neurostimulation. The rationale for the first approach is that essential tremor and Parkinson's disease are primarily caused by the brain structures that exhibit an abnormal oscillatory activity. Although a detailed description of such networks is still lacking, it is accepted that the desynchronization of this abnormal activity is the mechanism that mediates tremor management with deep brain stimulation, and also likely, through pharmacotherapy. In addition, the delivery of afferent stimuli to the supraspinal centers from the periphery has recently been shown to improve the symptoms of Parkinson's disease. The neurostimulator defined in this patent will directly stimulate the nerves innervating the tremulous muscles. This stimulation will be reaching the central tremorogenic network without interfering with other neural processes. To this end, the neurostimulator will stimulate in a range of frequencies aimed at causing a desynchronization of the tremor network.
- The second strategy is the decorrelation of the common input to motor neurons that innervate the affected muscles. This approach relies on the hypothesis that the desynchronization of the common tremor input to the motor neuron pool may reduce tremor amplitude. The rationale for this is that a secondary input, commonly projected to a pool of motor neurons, hampers the transmission of a primary input - in this case the tremor. Indeed, this technique is based on today treatments known to alleviate the symptoms of Parkinson's disease by vibrating the whole body. Interestingly, the experiments showing that limb cooling reduces the severity of tremor arising from Parkinson's disease, essential tremor, and multiple sclerosis, also provide support of this approach.

Furthermore, by using an Internet connection, the neuroprosthetic device can send objective information for the physician to monitor in real time (online) the effects of treatment for tremor suppression.

The main advantages of the method and device objects of this invention compared to other techniques and devices within the state of the art could be summarized as:
- It is a portable and ambulatory.
- It is less invasive than DBS and the percentage of eligible patients is higher.
- It is the first technique based on the afferent stimulation for minimizing the tremor.
- The concept proposed in this patent will replace or complement the pharmacological treatment of tremor.
- The device will allow monitoring tremor of the patient under any treatment, which could be based on the neuroprosthetic device described in this document or conventional treatment (pharmacotherapy or surgery).
- It does not limit the range of movements and freedom of the patient.

In addition, the invention relates to the following aspects.
1.- Neuroprosthetic device for monitoring and suppression of pathological tremor in a user via stimulation of the afferent pathways, characterized by comprising at least one wearable element located on the user, wherein the wearable element comprises:
   - At least one bioelectrical sensor that generates a bioelectrical characterization signal of the tremor;
   - At least one biomechanical sensor that generates a biomechanical characterization signal of the tremor;
   - A programmable electronic device comprising at least one control, acquisition and processing module for receiving and analyzing the bioelectrical and the biomechanical characterization signals, an electric signal generator for generating a signal of the afferent nerve stimulation based on the bioelectric and biomechanical characterization signals defined previously and,
   - At least one stimulation electrode integrated in the wearable element that generates the neuromodulation of the afferent pathways.
2.- Neuroprosthetic device according to aspect 1, characterized by comprising at least one EEG sensor which measures brain activity and generates a electroencephalography signal which characterizes intended voluntary movement of the user with the body part suffering from tremor.
3.- Neuroprosthetic device according to aspect 2, characterized by comprising an external device connectable to a programmable electronic device that implements an interface for accessing the functionalities of the at least one electroencephalography sensor and the wearable element.
4.- Neuroprosthetic device according to aspect 1, wherein at least one stimulation electrode is an electrode selected from an implanted electrode and a transcutaneous electrode.
5.- Neuroprosthetic device according to aspect 1, wherein the at least one bioelectrical sensor is selected between inertial sensors, sensors of conventional and high resolution electromyography, electroencephalography sensors measuring brain activity and characterizing the user intention to move or a combination of those.
6.- Neuroprosthetic device according to aspect 5, in which the inertial sensors are selected from gyroscopes, accelerometers, magnetometers and a combination of those.
7.- Neuroprosthetic device according to aspect 5, in which the electromyography sensors are multichannel thin film electrodes.
8.- Neuroprosthetic device according to any of the preceding aspects, wherein at least one stimulation electrode is a thin film multi-channel electrode.
9.- Neuroprosthetic device according to aspect 3, wherein the programmable electronic device is selected from a computer, a smartphone and a tablet device.
10.- Neuroprosthetic device according to any of the aspects 3 or 9, wherein the connection between the programmable electronic device and the external device is selected from a wireless connection or a wired connection.
11.- Neuroprosthetic device according to any of the aspects 3, 9 and 10, wherein the external device comprises means for displaying measurements taken by the neuroprosthetic diagnostic device based on the available sensors, a processor for analysis of tremor and generation of diagnostics and reporting, and a memory storage for the measurements made by the neuroprosthetic device.
12.- Method for monitoring and suppression of pathological tremor via the stimulation of the afferent pathways to the brain of the user, making use of the device described in any one of the aspects 1 to 11, characterized by comprising the following stages:
   i) characterizing the tremor by means of a plurality of bioelectric and biomechanical sensors, generating a bioelectrical and biomechanical characterization signal of the tremor wherein the bioelectrical and biomechanical characterization signals comprises at least the instantaneous frequency, phase or amplitude of the tremor sending these bioelectrical and biomechanical characterization signals to the control, acquisition and processing module of the programmable electronic device;
   ii) differentiating the tremor from the voluntary movement of the user through a conventional analysis of the bioelectrical and biomechanical characterization signals by the control, acquisition and processing module;
   iii) calculating a neurostimulation signal from the signal characterization of tremor in the control, acquisition and processing module of the programmable electronic device;
   iv) generating the neurostimulation signal through the signal generator module of the programmable electronic device and,
   v) To generate the neurostimulation signal to the plurality of stimulation electrodes activating the afferent pathways.
13.- Method for monitoring and suppression of pathological tremors according to aspect 12, characterized in that when the device has at least one EEG sensor which measures brain activity of the user, the method comprises the following steps:
   i) measuring, additionally to step i) the user's brain activity by at least one EEG sensor and generating the electroencephalography signal that characterizes the intention of the user to perform a voluntary movement of the limb affected by tremor;
   ii) sending, additionally in Phase II), the electroencephalography signal to the control, acquisition and processing module of the programmable electronic device;
   iii) To distinguish, additionally in Phase III), the tremor from the voluntary movement of the user through a conventional analysis of the electroencephalography signal, and,
   iv) To additionally employ EEG signal in step iv) to calculate the neurostimulation signal.
14.- Method for monitoring and suppression of pathological tremors, according to aspects 12 or 13, wherein the neurostimulation signal generate an afferent input flow to the central nervous system that disrupts the low-frequency synchronization in the corticobasal ganglia circuits.
15.- Method for monitoring and suppression of pathological tremors according to any preceding aspect, wherein the conventional analysis employed by the programmable electronic device for differentiating the tremor from the voluntary movement is based on algorithms in the time and frequency domain.

### DETAILED DESCRIPTION OF THE FIGURES

Figure 1. - Illustrates schematically the architecture of a particular implementation of the neuroprosthetic device defined in the present invention.
Figure 2. - Illustrates an example implementation of the device object of the invention placed on a limb of a user.
Figure 3. - Illustrates an example implementation of the method of monitoring and suppression of pathological tremors defined in the present invention.

### EXAMPLES

This section provides a description of several implementations of the invention, with an illustrative and non-limiting approach, making reference to the numbering adopted in the figures.

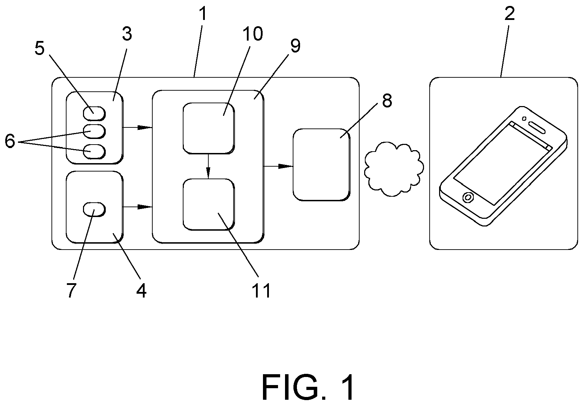

Figure 1 shows an implementation of the neuroprosthetic device composed of the wearable garment (1), in this case textile, and the connectable external device (2) from where the operation of the neuroprosthesis and the tremor characteristics are monitored.

This neuroprosthesis, which will be useful for monitoring, diagnosis and pathological tremor suppression in any of its variants, is being composed of the following components:
▪ Wearable garment (1) that enables the integration of inertial sensors, and electronics for neurostimulation and electrophysiological signal acquisition and processing, together with a module for data storage and communication. This item (1) provide long-term records of neural and biomechanical data of the body part to which it is placed, and will treat the tremor during the patient's daily life by delivering the afferent stimulation. The collected information is analyzed, and derived metrics are extracted to provide both the patient and clinician with feedback of the therapy progress. The clinician will have remote access to this data, and will be able to adjust the therapy, and the user will be involved in the treatment.
▪ Bioelectric sensors (3):
   a. Electrodes to measure surface (EMG) or intramuscular (iEMG) electromyography (6). These may be bipolar or high-density electrodes (6).
   b. Electrodes to measure electroencephalography (EEG) (5) .
   The information from the bioelectric sensors may be useful for the management of control strategies for neurostimulation.
▪ Motion sensors (4) such as inertial sensors (IMUs) (7).
▪ Electrodes (transcutaneous or implantable) for neurostimulation (8).
▪ A programmable electronic device (9) which may be a microcontroller, microprocessor, DSP, ... computer, which is responsible for:
   a. The identification and tracking of the tremor motion using appropriate state of the art algorithms.
   b. The implementation of control algorithms for neurostimulation.
   c. The acquisition of the sensors signals (3, 4) of the wearable element (1).
   d. The generation of signals for neurostimulation.
   e. Communication with an external device (2). The programmable electronic device (9) is able to communicate both wirelessly (Bluetooth, Zigbee, WiFi or IrDA) or through cables (TCP / IP, serial cable) with the external device (2).
▪ An external device (2) (PC, iDevice (iPhone, iPad), Smartphone or similar) responsible for:
   - The implementation of an interface to access all the features of the neuroprosthesis. This interface allows:
      i. The adjustment of the parameters of the control strategies for neurostimulation.
      ii. The definition of the operation mode of the neuroprosthesis.
      iii. The presentation of the variables measured by the neuroprosthesis.
      iv. The analysis of the tremor motion.
      v. The presentation of the machine-based diagnostic.
      vi. The storage of the data acquired by the neuroprosthesis.
      vii. The preparation of reports.
   - The communication (wirelessly or via cables) with the neuroprosthesis.

For the case in which the electrodes for neurostimulation are implantable (8), all the electronics would be integrated into the textile and only the electrode (intramuscular thin film or wire) would be implanted in the muscle. For the case in which the electrodes for neurostimulation are transcutaneous (8), all the electronics and the electrodes would be integrated into the textile.

The programmable electronic device (9) comprises the entire electronic for control, acquisition and processing of the signals acquired by the sensors. The electronic device (9) is also running different calibration strategies for the control and suppression of tremor and it is capable of generating the appropriate signal for the afferent neurostimulation based on the information gathered by the sensors (3,4) and it is also able to communicate with the external device (2). This external device (2), which is connected to the programmable electronic device (9), is able to provide an interface to access all the features of the neuroprosthesis (1) and present data in a convenient way to the users, who could be a patient or a clinician.

The sensors, biomechanical and bioelectrical (3, 4), would be used to extract information about the patient's movement and, from the algorithms programmed into the neuroprosthesis, extract features of patient's tremor. These tremor features allow customization of stimulation applied to each patient.

The use of this neuroprosthetic device is twofold. On one hand, it can be used as a tool for tremor evaluation and objectification in patients under normal conditions or under any tremor suppression treatment (pharmacological or surgical) and, on the other hand, as a tool for treatment of these patients with tremors.

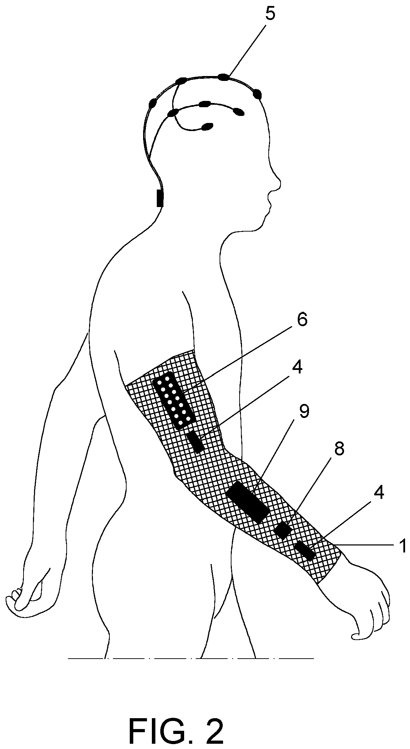

Figure 2 shows another implementation of the neuroprostetic device object of the present invention in which the wearable garment is placed on the arm and forearm of a user. Therefore the wearable element (1), which in this case will be textile, integrates bioelectrical sensors (3), movement sensors (4), a programmable electronic device (9) and neurostimulation electrodes (8).

This example is based on the adoption of two gyroscopes as motion sensors (4). These sensors are integrated into a flexible substrate, and are therefore very convenient for the integration within the garment. Both gyros are placed on the textile structure on both sides of the wrist joint and provide information about the absolute angular speed of the segments (hand and forearm) in the plane of motion of the joint. The sensors measure the frequency range between 0 and 50 Hz, thereby covering the entire frequency range of possible tremors.

Bioelectric sensors (3) used in the example for the measurement of bioelectrical activity are the high-density multichannel thin film iEMG electrodes. Electroencephalography (5) (EEG) sensors are also used and are responsible for detecting the user's voluntary intention to move.

In the example, implantable thin film electrodes (8) are used for neurostimulation and are responsible for the generation of electric fields that modulate the upper limb afferent pathways, projecting the afferent inflow to spinal and supraspinal centers, thereby causing the interruption of the low frequency synchronization in corticobasales ganglia circuits and, consequently, the suppression of tremor.

All algorithms used for cancellation of tremor could be implemented using programming environments that are suitable for the development of real time systems (e.g., C++). The algorithms are implemented in a microcontroller (integrated in the programmable electronic device (9)) capable of capturing all the sensor signals. In this particular example the acquisition rate is 200 kHz and the resolution 12 bits. This microcontroller also incorporates a Bluetooth module for wireless communication with the external device (2), not shown in the Figure.

The strategy for identifying and monitoring the tremor is based on the signals from the sensors (bioelectrical (3) and biomechanical (4)). The EEG signal sensors provide the information about the patient intent to move voluntarily by integrating two Bayesian classifiers that are based on different characteristics of the event related desynchronization (ERD) in EEG signals. EMG sensors indicate the onset of tremor and finally, the amplitude and frequency of the tremor are extracted from the inertial sensor data. The sampling frequency chosen for the loop identification is 1 KHz.

As external device (2) responsible for the implementation of the user interface is based on an iPad. The user interface is programmed into the iOS operating system. The communication between the programmable electronic device (9) and the external device (2) is made via Bluetooth.

The platform in this example provides long-term records of neural and biomechanical data of upper limb tremor. The tremor is treated during the patient's daily life by afferent nerve stimulation. The collected information is analyzed, and metrics are derived in order to provide the feedback about the performance of the therapy to the patient and the clinician. The remote medical professional has remote access to these data via the interface programmed in the iPad, and is able to adjust the therapy. As a result, the user is more involved in the treatment.

The final product will thus be an aesthetically appealing wearable neuroprosthesis that will integrate all the electronics for control, acquisition and processing, and the flexible inertial sensors on a textile substrate, together with a set of implantable thin film electrodes for neurostimulation and neural recordings. The neuroprosthesis constitutes an alternative management of tremor, either alone or in combination with pharmacotherapy.

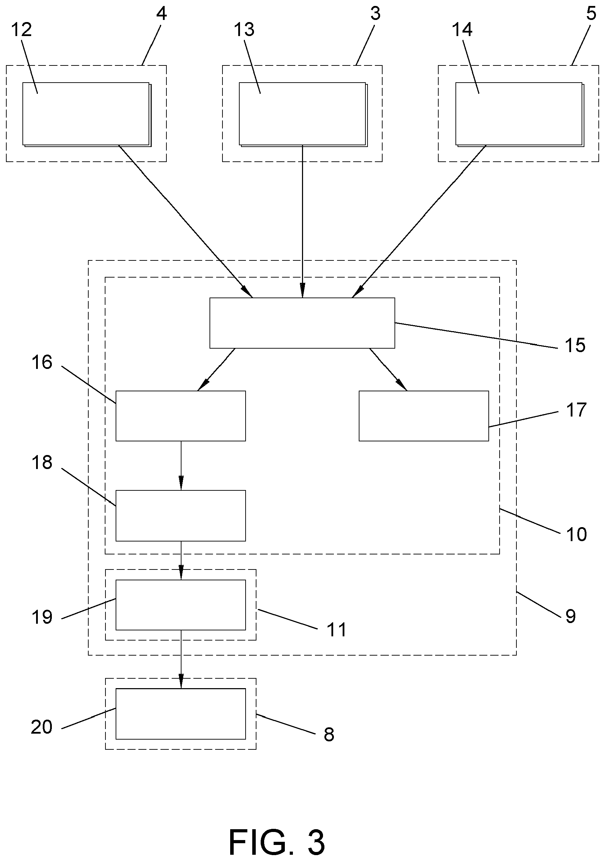

Figure 3 shows a particular example of the method for monitoring and suppression of pathological tremors in which biomechanical characterization signals (12), captured by the inertial sensors (4), bioelectrical characterization signals (13), captured by the electromyography sensors (3) and electroencephalography sensors (14) are sent to the programmable electronic device (9) which differentiate (15) the tremor (16) from the voluntary movement (17) of the user. This operation is performed in the control acquisition and processing module (10) of the electronic device (9). From the characterization of tremor signal (16) the control module (10) calculates (18) the necessary neurostimulation to compensate the tremor (16). This signal is sent to neurostimulation signal generator (11), generating (19) this signal and sending it to the neurostimulation electrodes (8) that eventually generate (20) the afferent inflow to the central nervous system.

## Claims

1. A transcutaneous wearable system for monitoring and suppression of a pathological tremor in a user via stimulation of afferent pathways, the system comprising:
at least one biomechanical sensor;
wherein the biomechanical sensor is configured to generate a biomechanical characterization signal of the pathological tremor;
at least one bioelectronic sensor;
wherein the bioelectronic sensor is configured to generate a bioelectronic characterization signal of the pathological tremor;
a programmable electronic device for receiving and analyzing the biomechanical characterization signal and the bioelectronic characterization signal to determine different calibration strategies for controlling and suppressing the pathological tremor; and
an electric signal generator for generating a signal of afferent nerve stimulation, via at least one transcutaneous stimulation electrode, based at least in part on the biomechanical characterization signal and based at least in part on the bioelectronic characterization signal;
wherein the at least one transcutaneous stimulation electrode is integrated into an external wearable element,
wherein the external wearable element is configured to at least partially encircle a limb,
wherein the programmable electronic device is configured to communicate information to an external device from a user related to the different calibration strategies of the nerve stimulation, and
wherein the programmable electronic device is further configured to receive adjustments to the therapy from the external device based on the different calibration strategies.

2. The transcutaneous wearable system according to Claim 1, wherein the different calibration strategies relate to at least one of the biomechanical characterization signal or the signal of afferent nerve stimulation.

3. The transcutaneous wearable system according to any of the preceding claims, wherein the biomechanical characterization signal comprises at least frequency, phase or amplitude of the pathological tremor.

4. The transcutaneous wearable system according to any of the preceding claims, wherein the biomechanical sensor is configured as an inertial measurement unit.

5. The transcutaneous wearable system according to any of the preceding claims, wherein the programmable electronic device is a computer, a smartphone or a tablet device.
